# EUROPEAN PATENT SPECIFICATION

(11) **EP 3 982 923 B1**
(45) Date of publication and mention of the grant of the patent: **15.05.2024**
(21) Application number: 20734291.6
(22) Date of filing: 16.06.2020
(51) Int. Cl.: A61K 9/00, A61K 31/439, A61K 31/46, A61P 11/06, C07D 453/02

(54) **CONTINUOUS PROCESS FOR THE PREPARATION OF ANTICHOLINERGIC DRUGS**
KONTINUIERLICHES VERFAHREN ZUR HERSTELLUNG VON ANTICHOLINERGEN ARZNEIMITTELN
PROCÉDÉ CONTINU POUR LA PRÉPARATION DE MÉDICAMENTS ANTICHOLINERGIQUES

(30) Priority: 17.06.2019 PT 2019115583
(43) Date of publication of application: 20.04.2022
(73) Proprietor: Hovione Scientia Limited, Ringaskiddy, Co. Cork (IE)
(72) Inventor: KATZ, Marianna, 1750-014 Lisbon (PT); TORRES LOURENÇO, Nuno, 1750-014 Lisbon (PT)
(74) Representative: Paulraj, Leonita Theresa
(86) International application number: PCT/GB2020/051448
(87) International publication number: WO 2020/254791

(56) References cited:
- WO-A1-2014/027045
- WO-A1-2018/087561
- US-B2- 8 865 903

## Description

### FIELD OF THE INVENTION:

The present invention relates generally to a novel process for the preparation of anticholinergic agents/drugs, such as umeclidinium bromide (I) (chemical name: 4-[hydroxyl(diphenyl)methyl]-1-[2-(phenylmethyl)oxy]ethyl]-1-azoniabicyclo[2.2.2]octane bromide), tiotropium bromide (II) (chemical name: 1α,2β,4β,7β)-7-[(hydroxy-di-2-thienylacetyl)oxy]-9,9-dimethyl-3-oxa-9-azonia-tricyclo[3.3.1.0^{2,4}]nonane bromide), glycopyrronium bromide (III) (chemical name: 3-(2-cyclopentyl-2-hydroxy-2-phenylacetoxy)-1,1-dimethylpyrrolidinium bromide), aclidinium bromide (IV) (chemical name: 3(R)-(2-hydroxy-2,2-dithien-2-ylacetoxy)-1-(3-phenoxypropyl)-1-azoniabicyclo [2.2.2] octane bromide), ipratropium bromide (V) (chemical name: [8-methyl-8-(1-methylethyl)-8-azoniabicyclo[3.2.1]oct-3-yl]-3-hydroxy-2-phenyl-propanoate) in continuous flow mode, and to the agents so prepared, and to their use in medicine. The anticholinergic drugs listed above are used in the treatment of respiratory diseases such as asthma or chronic obstructive pulmonary disease (COPD).

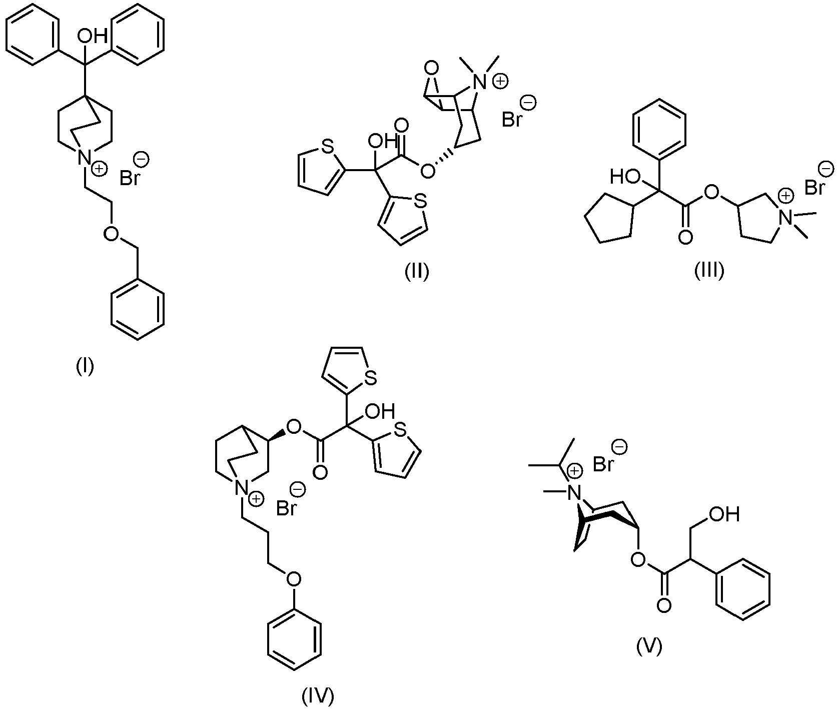

### BACKGROUND OF THE INVENTION:

The compounds of molecular structures (I-V) depicted above are known-anticholinergic drugs with different applications, mainly used for the treatment of chronic obstructive pulmonary disease (COPD) and asthma.

Umeclidinium bromide is a highly effective active pharmaceutical ingredient used for preparing pharmaceutical compositions to be administered as a dry powder for oral inhalation at once-daily micrograms dose. New compositions, combinations, forms of administration (e.g. metered-dose inhalers) and dosages using umeclidinium bromide are being developed.

WO 2005/104745 relates to a process for the preparation of umeclidinium bromide, wherein the reaction is carried out in chlorinated solvents for a time period of between 16 hours and 24 hours:

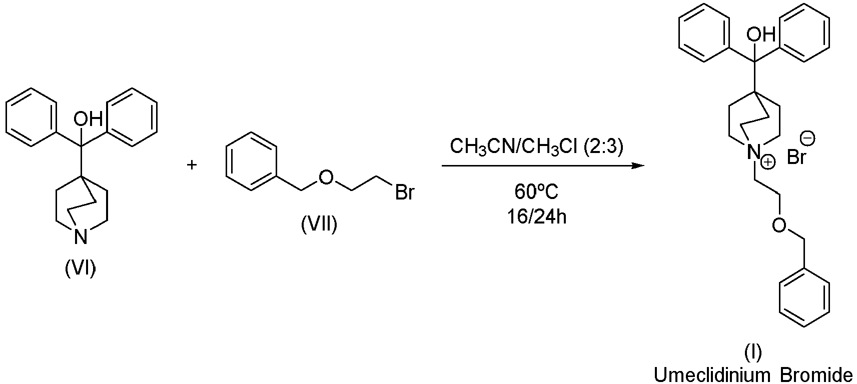

Unsolvated crystalline polymorphic forms of umeclidinium bromide have been disclosed (WO 2014/027045, US 9273001 B2) as the active pharmaceutical ingredient showing that the compound may give rise to a variety of crystalline solid state form having distinct physical properties. The preparation of pure umeclidinium bromide in a single, pure crystalline form with a consistent level of crystallinity and chemical purity has been a challenge to the industry due to the compound's high susceptibility to form solvates. Umeclidinium bromide solvates, namely methanol solvate (CZ27764 (Sanofi)), ethanol, 2-propanol, 2-methylpropan-1-ol, chlorobenzene and p-xylene solvates (WO 2014/027045, US 9657011 B2) have been identified. In the batch preparation of umeclidinium bromide 1-propanol has been used as solvent to minimize the solvate formation (WO 2014/027045, US 9657011 B2) avoiding the resuspension of the compound in ethyl acetate, methanol and water, which was previously required (example 84, Method B, WO 2005/104745). In the batch preparation of umeclidinium bromide a thick slurry is formed, which is cooled and aged for a few to several hours and then filtered and washed with n-proposal to obtain the product (WO 2014/027045, US 9657011 B2).

EP3248970 relates to solid forms of umeclidinium bromide, in particular to its non-solvated form 1, crystalline forms A and B and an amorphous form. Form A is prepared by crystallization from a solution of umeclidinium bromide in a mixture of methanol and water in the volume ratio of 1:1 to 2:1. Form B is obtained from form A at a temperature higher than 180°C.

WO 2018/087561 relates to a process for the preparation of umeclidinium bromide having high purity, wherein the reaction is carried out for a time period of between 18 h to 24 h, the solvent is selected from cyclic ethers such as tetrahydrofuran, aromatic solvents, such as toluene, ketones such as acetone and protic solvents such as water or combinations thereof, to form a product with a single, pure crystalline form with a consistent level of crystallinity and chemical purity.

Tiotropium bromide is a highly effective active pharmaceutical ingredient which is administrated in low (microgram) therapeutic doses by inhalation. Crystalline polymorphic forms of tiotropium bromide have been reported in various publications, including US6777423, EP14101445, EP16825442, EP1879888, EP2085396, EP1869035, and WO2011/01588, showing that the compound may give rise to a variety of solid forms having distinct physical properties.

EP2814827 describes a process where tiotropium bromide is obtained by crystallization in a mixture comprising methanol and acetone, in a single pure anhydrous crystalline form.

RU2453547 provides a crystalline hemi-n-propanol solvate of tiotropium bromide, designated as Form 12, prepared by crystallization from a solution of tiotropium bromide in n-propanol.

Against the backdrop of stringent regulations, pharmaceutical companies are focusing on developing new methods to provide more efficient technology to meet the demand in highly competitive pharmaceutical and healthcare industry. Flow chemistry techniques and systems have witnessed tremendous growth in the pharmaceutical industry in recent years. The pharma sector is focusing on improving existing chemical reactions and accessing new chemicals with the help of flow chemistry.

With increasing competition in the pharmaceutical sector, companies are concentrating on rapid development, seamless discovery, and optimizing potential drug compounds, thereby reducing time to market are some of the key driving factors leading to the rapid adoption of flow chemistry.

US8865903 relates to a continuous flow process for the preparation of quaternary ammonium salts, such as tiotropium bromide (II), glycopyrronium bromide (III) or ipratropium bromide (V) with high yield and purity in polar aprotic solvents selected from the group consisting of amides, nitriles and sulphoxides such as acetonitrile, dimethylformamide, dimethylacetamide, N-methylpyrrolidone and dimethylsulfoxide. The invention describes that dichloromethane is a poor solvent for the final product, which may precipitate in the continuous-flow reactor and obstruct the channels. US8865903 teaches that the replacement of dichloromethane with methanol allows to increase the temperature, but conversion is rather low.

In order to fulfill the market demand, the present inventors have recognized there exists a need for a more efficient process for preparing anticholinergic drugs. In particular, a process that offers advantages over the known processes for the preparation of anticholinergic agents. The inventors have now devised such a process. The advantages of the present invention include, but are not limited to, a very efficient process with reduced reaction time, improved operability, temperature control, yield and a reduced cost of the final product, which is critical to optimize any synthetic process. Notably, even a small improvement in reaction design can lead to significant savings in a large scale production.

Consequently, the present invention discloses a continuous flow process for the preparation of anticholinergic drugs, such as umeclidinium bromide, tiotropium bromide, glycopyrronium bromide, aclidinium bromide or ipratropium bromide, in one or more polar protic solvents, preferably 1-propanol, water or a mixture of 1-propanol and water to form a single, pure crystalline form with good yield and excellent chemical purity.

### SUMMARY OF THE INVENTION:

According to the present invention, there is provided a process for the preparation of an anticholinergic agent, wherein the process is carried out in continuous flow mode using a solvent consisting of one or more polar protic solvents.

According to the present invention, there is provided a process for the preparation of one or more anticholinergic drugs, such as umeclidinium bromide, tiotropium bromide, glycopyrronium bromide, aclidinium bromide or ipratropium bromide in one or more polar protic solvents such as 1-propanol, water or a mixture of 1-propanol and water, in continuous flow mode.

It has been found that contrary to the state of the art (US8865903) where the inventor describes only polar aprotic solvents are adequate for the continuous process approach in the preparation of quaternary ammonium salts, such as tiotropium bromide (II), glycopyrronium bromide (III) or ipratropium bromide (V), the inventors of the present invention have surprisingly found that one or more polar protic solvents, such as 1-propanol, water or a mixture of 1-propanol and water, used in a continuous flow mode process provided an efficient continuous process of preparing anticholinergic drugs without the need for additional resuspension or recrystallization step that is required in the prior art process, at a very reduced reaction time.

The inventors of the present invention have found that the reaction time of the process disclosed in the present invention is extremely reduced from 16-24 hours to 1-20 minutes. The anticholinergic agent obtained using the novel process described in the present invention may be a single, pure solid crystalline form with a consistent level of crystallinity and chemical purity.

The anticholinergic agents obtained from the process of the present invention can be formulated, preferably with at least one pharmaceutically acceptable excipient, wherein the composition is suitable for inhalation. Preferably, the pharmaceutical composition comprises the anticholinergic agent obtained by the process of the present invention and one or more additional active pharmaceutical ingredients. Preferably, the pharmaceutical composition may be in the form of a dry powder, a solution or a suspension.

### DETAILED DESCRIPTION OF THE INVENTION:

The present invention provides a process for the preparation of an anticholinergic drug, in continuous flow mode in the presence of one or more polar protic solvents.

The anticholinergic drug disclosed in the present invention, includes, but not limited to, umeclidinium bromide, tiotropium bromide, glycopyrronium bromide, aclidinium bromide and ipratropium bromide.

According to another aspect of the present invention, there is provided a process for the preparation of umeclidinium bromide comprising reacting 1-azabicyclo[2.2.2]oct-4-yl(diphenyl)methanol (VI) with ((2-bromoethoxy)methyl)benzene (VII) in one or more polar protic solvents in continuous flow mode.

Any polar protic solvent that do not form a solvate with the anticholinergic drug disclosed in the present application may be used in the process of the present invention.

The one or more polar protic solvents used in the process disclosed in the present invention, includes, but not limited to, 1-propanol, water, a mixture of 1-propanol and water, or any isomer of propanol or butanol, such as n-propanol, n-butanol.

Preferably, the ratio of 1-propanol and water in the mixture of 1-propanol and water is in the range of from about 30:1 to 1:1.2, preferably about 25:1.

A skilled person can determine the amount of starting material or chemicals, such as cyclic tertiary amines, alkylating agents, to be used in the process of preparing an anticholinergic drug disclosed in the present invention. For example, in the process of preparing umeclidinium bromide disclosed in the present invention, the concentration of 1-azabicyclo[2.2.2]oct-4-yl(diphenyl)methanol (VI) to be used is dependent on the solubility of the compound at a specific temperature at which the reaction is carried out. Preferably, a concentration in the range of from about 1 to about 10 mol/L equivalent of ((2-bromoethoxy)methyl)benzene (VII) may be used in the process.

Preferably, the novel process of the present invention requires no further resuspension or recrystallization of the drug to obtain an unsolvate form.

The process for the preparation of the anticholinergic agent/drug disclosed in the present invention may comprise using one or more flow procedures to carry out the continuous flow mode process.

The term "flow procedures" as used herein relates to those procedures, for example, the use of certain apparatus and/or certain conditions, necessary to enable the continuous running of chemical synthesis. Flow procedures, as used herein, does not encompass a traditional batch process. Preferably, a continuous reactor is used to carry material as a flowing stream, as will be understood by those skilled in this field.

A process may be defined as continuous in that it is characterized by continuous feeding of the reactants to the reactor with continuous formation and exiting of a product stream.

A continuous process disclosed in the present invention may be advantageous for a number of reasons including, but not limited to, improved purity and yield of the product and reduced effluent; thus, making the present process more environmentally friendly.

The term "continuous flow reactor" is used to refer to those reactors which enable chemical reactions to occur in a continuous flow. Continuous flow reactors may also be known as continuous tubular reactors. The continuous flow reactor may comprise a pipe reactor, a plug flow reactor, a tube reactor or another commercially available continuous flow reactor, or a combination of two or more such reactors.

In the process for the preparation of the anticholinergic agent/drug disclosed in the present invention, the starting materials or chemicals such as cyclic tertiary amines, alkylating agents, is preferably in the form of a solution dissolved in a suitable solvent, such as the polar protic solvents disclosed in the present invention and fed continuously into a continuous-flow reactor.

In the process of preparing umeclidinium bromide disclosed in the present invention, a solution comprising 1-azabicyclo[2.2.2]oct-4-yl(diphenyl)methanol (VI) and ((2-bromoethoxy)methyl)benzene (VII) is prepared, preferably in a polar protic solvent such as 1-propanol, water or in a mixture of 1-propanol and water. The solution of 1-azabicyclo[2.2.2]oct-4-yl(diphenyl)methanol (VI) and ((2-bromoethoxy)methyl)benzene (VII) is introduced into a continuous flow reactor separately or, as an alternative, the solutions comprising the reactants can be pre-mixed before the introduction into the continuous flow reactor.

The temperature under which the reaction in the polar protic solvent, such as 1-propanol, water or a mixture of 1-propanol and water is carried out, is preferably in the range of from about 120 °C to about 200 °C. Preferably, the reaction temperature is above 140 °C. Preferably, the reaction temperature is in the range of from about 140 °C to about 180 °C. Preferably, the reaction temperature is in the range of above 140 °C to 180 °C. More preferably, the reaction temperature is in the range of from about 141 °C to about 180 °C. Most preferably, the reaction temperature is in the range of from about 150 °C to 180 °C.

Surprisingly, despite the extreme temperatures used in the process of the present invention no significant impurity formation or degradation was observed between the polar protic solvent (water or/and 1-propanol), and the alkylating agent, such as ((2-bromoethoxy)methyl)benzene in the case of the preparation process of umeclidinium bromide of the present invention.

Surprisingly, the inventors also found that use of polar protic solvent such as 1-propanol, water or a mixture of 1-propanol and water, in continuous flow mode process of the present invention, at extreme temperatures specified above resulted in reduced reaction time, good yield and high purity.

The inventors of the present invention have found that the reaction time of the process disclosed in the present invention is extremely reduced from 16-24 hours to 1-20 minutes. Consequently, the reaction time of the process of the present invention is preferably in the range of from about 1 to about 20 minutes, preferably the reaction time of the process of the present invention is in the range of from about 2 to about 20 minutes, more preferably the reaction time of the process of the present invention is in the range of from about 5 to about 20 minutes, most preferably the reaction time of the process of the present invention in the range of from about 5 to about 10 minutes.

The flow rate may be adjusted in order to obtain an optimal residence time of the reaction mixture in the continuous flow reactor with the aim of completing the reaction. The flow rate of reaction mixture through the one or more continuous flow reactors may be controlled, altered or adjusted depending on the chemical reaction to be carried out, and the reactor model being used.

Flow and pressure ranges used are characteristics of the reaction model. A skilled person can determine the flow rate and pressure ranges depending upon the reactor model being used. For example, in the case of a custom-made stainless steel coil reactor, typically the flow is in the range of from about 0.11 to about 0.84 mL/min and the pressure is in the range of from about 3 to 34 about bar.

Preferably, in the process disclosed in the present invention, the product is isolated by removing the solvent from the reaction mixture. The reaction mixture obtained from the reactor may be concentrated under reduced pressure. An anti-solvent, preferably water may be added to the concentrated product to form an aqueous suspension. The aqueous suspension may be cooled down to a temperature, preferably in the range of from about 0 °C to about 5 °C or from above 0 °C to about 5 °C, and thereafter the solid crystalline form of the product is isolated, preferably by filtration. The filtered product may be washed and/or dried, preferably under reduced pressure to obtain the product in yields up to 80%. The purity of the product obtained by following the procedure described in the present invention is in a single crystalline form typically ≥98.5% by HPLC. The product obtained is preferably umeclidinium bromide.

The X-Ray Powder Diffraction (XRPD) diffractogram, the Differential Scanning Calorimetry (DSC) thermogram, the Thermogravimetric Analysis (TGA) thermogram and HPLC chromatogram used for analyzing the product obtained are in accordance with the state of the art.

In a particularly preferred embodiment, the process disclosed in the present invention is useful for the preparation of umeclidinium bromide.

The anticholinergic agents, such as umeclidinium bromide, tiotropium bromide, glycopyrronium bromide, aclidinium bromide or ipratropium bromide obtained by the process disclosed in the present invention may be further micronized to obtain material with adequate particle size which is suitable for inhalation.

Not encompassed by the wording of the claims are anticholinergic agents obtained by the claimed process, pharmaceutical compositions comprising said anticholinergic agents, and said anticholinergic agents or said pharmaceutical compositions for use as a medicament, preferably for use in treating diseases such as asthma or chronic obstructive pulmonary disease (COPD).

The present disclosure also provides an anticholinergic drug, such as, umeclidinium bromide, tiotropium bromide, glycopyrronium bromide, aclidinium bromide or ipratropium bromide obtained by the novel process of the present invention. In a preferred embodiment of the present disclosure, the anticholinergic drug is umeclidinium bromide.

Preferably, the anticholinergic drugs are in a form suitable for inhalation. The crystalline form of the anticholinergic drugs obtained by the process of the present invention may be in an unsolvated form.

The present disclosure also provides a pharmaceutical composition comprising an anticholinergic drug disclosed herein, preferably umeclidinium bromide obtained by the process of the present invention and at least one pharmaceutically acceptable excipient. The pharmaceutical composition is suitable for inhalation, preferably in the form of a dry powder, a solution or a suspension. The pharmaceutical composition of the present disclosure may further comprise an additional one or more active pharmaceutical ingredients.

The one or more active pharmaceutical ingredients used in the pharmaceutical composition disclosed in the present disclosure, includes, but not limited to, Vilanterol trifenatate, Fluticasone furoate or a combination therof.

The pharmaceutical compositions may be prepared by admixing an anticholinergic drug disclosed herein, preferably umeclidinium bromide obtained by the novel process of the present invention and one or more pharmaceutically acceptable excipients.

The pharmaceutical composition of the present disclosure is for use as a medicament, preferably for use in treating respiratory diseases such as asthma or chronic obstructive pulmonary disease (COPD).The present disclosure further provides a method for the treatment in a mammal, such as a human, for treating respiratory, inflammatory or obstructive airway disease such as COPD and asthma, which method comprises administration of a therapeutically effective amount of a pharmaceutical composition of the present disclosure.

The dosage and mode of administration can be decided by the expert of the art, based on the common general knowledge.

The pharmaceutical compositions may be formulated to be delivered by any suitable route, including oral, intravenous, parenteral, inhalation, intranasal, topical, subcutaneous, or intramuscular.

The pharmaceutical compositions of the present disclosure may be administered by any suitable method used for delivery of drugs to the respiratory tract. The composition of the present disclosure may thus be administered using metered dose inhalers (MDI), dry powder inhalers (DPI), nebulisers, nasal sprays, nasal drops, insufflation powders, sprays and spray patches.

The present invention is now illustrated without limiting it by the following examples.

### Example 1

### Preparation of umeclidinium bromide

A solution of 1-azabicyclo[2.2.2]oct-4-yl(diphenyl)methanol (0.3 g, 1.0 mmol) and ((2-bromoethoxy)methyl)benzene (0.24 mL, 1.5 mmol) in 1-propanol (30 mL) was injected into a stainless steel coil continuous flow reactor (2.1 mL) at rate of 0.11 mL/min. The reactor temperature was 180 °C. The reaction time was 20 minutes. The solution coming out from the continuous flow reactor was collected (conversion by HPLC: 93.8 %), concentrated to a volume of 4 mL under reduced pressure. The resulting suspension was cooled down to 5 °C and stirred for 1 hour. The product was filtered, washed twice with methyl tert-butyl ether (MTBE) and dried under reduced pressure (white powder, 0.34 g, 80 %). The product was analyzed by HPLC resulting in 98.5 % purity.

### Example 2

### Preparation of umeclidinium bromide

A solution of 1-azabicyclo[2.2.2]oct-4-yl(diphenyl)methanol (0.34 g, 1.2 mmol) and ((2-bromoethoxy)methyl)benzene (0.27 mL, 1.7 mmol) in 1-propanol (10 mL) was injected into a stainless steel coil continuous flow reactor (2.1 mL) at rate of 0.42 mL/min. The reactor temperature was 180 °C. The reaction time was 5 minutes. The solution coming out from the continuous flow reactor was collected (conversion by HPLC: 97.3 %), concentrated to a volume of 4 mL under reduced pressure. The resulting suspension was cooled down to 5 °C and stirred for 1 hour. The product was filtered, washed twice with methyl tert-butyl ether (MTBE) and dried under reduced pressure (white powder, 0.36 g, 75 %). The product was analyzed by HPLC resulting in 98.9 % purity.

### Example 3

### Preparation of umeclidinium bromide

A solution of 1-azabicyclo[2.2.2]oct-4-yl(diphenyl)methanol (0.3 g, 1.0 mmol) and ((2-bromoethoxy)methyl)benzene (0.24 mL, 1.5 mmol) in 1-propanol (30 mL) was injected into a stainless steel coil continuous flow reactor (2.1 mL) at rate of 0.42 mL/min. The reactor temperature was 180 °C. The reaction time was 5 minutes. The solution coming out from the continuous flow reactor was collected, diluted and analyzed by HPLC resulting in 93.2 % conversion.

### Example 4

### Preparation of umeclidinium bromide

A solution of 1-azabicyclo[2.2.2]oct-4-yl(diphenyl)methanol (0.3 g, 1.0 mmol) and ((2-bromoethoxy)methyl)benzene (0.24 mL, 1.5 mmol) in 1-propanol (30 mL) was injected into a stainless steel coil continuous flow reactor (2.1 mL) at rate of 0.21 mL/min. The reactor temperature was 150 °C. The reaction time was 10 minutes. The solution coming out from the continuous flow reactor was collected, diluted and analyzed by HPLC resulting in 88.5 % conversion.

## Claims

1. A process for the preparation of an anticholinergic agent, wherein the process is carried out in continuous flow mode using a solvent consisting of one or more polar protic solvents.

2. The process according to claim 1, wherein the anticholinergic agent is selected from the group consisting of: umeclidinium bromide, tiotropium bromide, glycopyrronium bromide, aclidinium bromide and ipratropium bromide.

3. The process according to claim 1 or 2, wherein the anticholinergic agent is umeclidinium bromide.

4. The process according to claim 3, comprising reacting 1-azabicyclo[2.2.2]oct-4-yl(diphenyl)methanol (VI) with ((2-bromoethoxy)methyl)benzene (VII) in the presence of the one or more polar protic solvents.

5. The process according to claim 4, wherein 1-azabicyclo[2.2.2]oct-4-yl(diphenyl)methanol (VI) and ((2-bromoethoxy)methyl)benzene (VII) in the form of a solution is fed continuously to one or more continuous flow reactors.

6. The process according to any one of the preceding claims, wherein the solvent is 1-propanol, water or a mixture of 1-propanol and water.

7. The process according to claim 6, wherein the ratio of 1-propanol and water in the mixture is in the range of from 30:1 to 1:1.2, preferably 25:1.

8. The process according to any one of claims 5 to 7, wherein the solution comprising 1-azabicyclo[2.2.2]oct-4-yl(diphenyl)methanol (VI) and ((2-bromoethoxy)methyl)benzene (VII) in the one or more polar protic solvents is fed to the continuous flow reactor separately, or the solutions are pre-mixed before feeding into the continuous flow reactor.

9. The process according to any one of the preceding claims, wherein the reaction temperature is in the range of from about 120 °C to about 200 °C, preferably above 140 °C.

10. The process according to any one of the preceding claims, wherein the reaction temperature is from above 140 °C to 180 °C, preferably from about 150 °C to 180 °C.

11. The process according to any one of the preceding claims, wherein the reaction time is in the range of from about 1 to about 20 minutes, preferably from about 5 to about 10 minutes.

12. The process according to any one of the preceding claims, wherein no further resuspension or recrystallization of the agent is required to obtain an unsolvate form.

13. The process according to any one of the preceding claims, further comprising isolating the anticholinergic agent, preferably by:
(i) forming a concentrate of the solution comprising the anticholinergic from the reactor, preferably by applying reduced pressure;
(ii) adding an antisolvent, wherein the antisolvent is preferably water to form an aqueous suspension;
(iii) cooling the aqueous suspension down to a temperature of from about 0 °C to about 5 °C; and
(iv) isolating the product in a solid crystalline form , preferably by filtration.

14. The process according to any one of the preceding claims further comprising micronizing the anticholinergic agent.

## Patentansprüche

1. Verfahren zur Herstellung eines anticholinergen Mittels, wobei das Verfahren in einem Durchflußmodus unter Verwendung eines Lösungsmittels, bestehend aus einem oder mehreren polaren protischen Lösungsmitteln, durchgeführt wird.

2. Verfahren nach Anspruch 1, wobei das anticholinerge Mittel ausgewählt ist aus der Gruppe bestehend aus: Umeclidiniumbromid, Tiotropiumbromid, Glycopyrroniumbromid, Aclidiniumbromid und Ipratropiumbromid.

3. Verfahren nach Anspruch 1 oder 2, wobei das anticholinerge Mittel Umeclidiniumbromid ist.

4. Verfahren nach Anspruch 3, umfassend das Umsetzen von 1-Azabicyclo[2.2.2]oct-4-yl(diphenyl)methanol (VI) mit ((2-Bromethoxy)methyl)benzol (VII) in Gegenwart des einen oder der mehreren polaren protischen Lösungsmittel.

5. Verfahren nach Anspruch 4, wobei 1-Azabicyclo[2.2.2]oct-4-yl(diphenyl)methanol (VI) und ((2-Bromethoxy)methyl)benzol (VII) in Form einer Lösung kontinuierlich einem oder mehreren Durchflußreaktoren zugeführt werden.

6. Verfahren nach einem der vorhergehenden Ansprüche, wobei das Lösungsmittel 1-Propanol, Wasser oder ein Gemisch aus 1-Propanol und Wasser ist.

7. Verfahren nach Anspruch 6, wobei das Verhältnis von 1-Propanol und Wasser in der Mischung im Bereich von 30:1 bis 1:1,2, vorzugsweise 25:1, liegt.

8. Verfahren nach einem der Ansprüche 5 bis 7, wobei die Lösung, die 1-Azabicyclo[2.2.2]oct-4-yl(diphenyl)methanol (VI) und ((2-Bromethoxy)methyl)benzol (VII) in dem einen oder den mehreren polaren protischen Lösungsmitteln umfasst, dem Durchflußreaktor separat zugeführt wird oder die Lösungen vorgemischt werden, bevor sie dem Durchflußreaktor zugeführt werden.

9. Verfahren nach einem der vorhergehenden Ansprüche, wobei die Reaktionstemperatur im Bereich von etwa 120 °C bis etwa 200 °C, vorzugsweise über 140 °C, liegt.

10. Verfahren nach einem der vorhergehenden Ansprüche, wobei die Reaktionstemperatur von über 140 °C bis 180 °C, vorzugsweise von etwa 150 °C bis 180 °C, beträgt.

11. Verfahren nach einem der vorhergehenden Ansprüche, wobei die Reaktionszeit im Bereich von etwa 1 bis etwa 20 Minuten, vorzugsweise von etwa 5 bis etwa 10 Minuten, liegt.

12. Verfahren nach einem der vorhergehenden Ansprüche, wobei keine weitere Resuspension oder Rekristallisation des Mittels erforderlich ist, um eine unlösliche Form zu erhalten.

13. Verfahren nach einem der vorhergehenden Ansprüche, ferner umfassend das Isolieren des anticholinergen Mittels, vorzugsweise durch:
(i) Bilden eines Konzentrats der Lösung, die das Anticholinergikum aus dem Reaktor umfasst, vorzugsweise durch Aufbringen von reduziertem Druck;
(ii) Hinzufügen eines Antilösungsmittels, wobei das Antilösungsmittel vorzugsweise Wasser ist, um eine wässrige Suspension zu bilden;
(iii) Abkühlen der wässrigen Suspension auf eine Temperatur von etwa 0 °C bis etwa 5 °C; und
(iv) Isolieren des Produkts in fester kristalliner Form, vorzugsweise durch Filtration.

14. Verfahren nach einem der vorangehenden Ansprüche, ferner umfassend das Mikronisieren des anticholinergen Mittels.

## Revendications

1. Procédé de préparation d'un agent anticholinergique, dans lequel le procédé est effectué en mode à flux continu au moyen d'un solvant constitué par un ou plusieurs solvants protiques polaires.

2. Procédé selon la revendication 1, dans lequel l'agent anticholinergique est choisi dans le groupe constitué par : le bromure d'uméclidinium, le bromure de tiotropium, le bromure de glycopyrronium, le bromure d'aclidinium et le bromure d'ipratropium.

3. Procédé selon la revendication 1 ou 2, dans lequel l'agent anticholinergique est le bromure d'uméclidinium.

4. Procédé selon la revendication 3, comprenant la réaction de 1-azabicyclo[2.2.2]oct-4-yl(diphényl)méthanol (VI) avec du ((2-bromoéthoxy)méthyl)benzène (VII) en présence des un ou plusieurs solvants protiques polaires.

5. Procédé selon la revendication 4, dans lequel le 1-azabicyclo[2.2.2]oct-4-yl(diphényl)méthanol (VI) et le ((2-bromoéthoxy)méthyl)benzène (VII) sous la forme d'une solution sont fournis en continu à un ou plusieurs réacteurs à flux continu.

6. Procédé selon l'une quelconque des revendications précédentes, dans lequel le solvant est le 1-propanol, l'eau ou un mélange de 1-propanol et d'eau.

7. Procédé selon la revendication 6, dans lequel le rapport entre 1-propanol et eau dans le mélange est dans la plage de 30:1 à 1:1,2, de préférence 25:1.

8. Procédé selon l'une quelconque des revendications 5 à 7, dans lequel la solution comprenant le 1-azabicyclo[2.2.2]oct-4-yl(diphényl)méthanol (VI) et le ((2-bromoéthoxy)méthyl)benzène (VII) dans les un ou plusieurs solvants protiques polaires est fournie au réacteur à flux continu séparément, ou les solutions sont prémélangées avant fourniture au réacteur à flux continu.

9. Procédé selon l'une quelconque des revendications précédentes, dans lequel la température de réaction est dans la plage d'environ 120 °C à environ 200 °C, de préférence supérieure à 140 °C.

10. Procédé selon l'une quelconque des revendications précédentes, dans lequel la température de réaction est de plus de 140 °C à 180 °C, de préférence d'environ 150 °C à 180 °C.

11. Procédé selon l'une quelconque des revendications précédentes, dans lequel le temps de réaction est dans la plage d'environ 1 à environ 20 minutes, de préférence d'environ 5 à environ 10 minutes.

12. Procédé selon l'une quelconque des revendications précédentes, dans lequel aucune autre remise en suspension ou recristallisation de l'agent n'est requise pour obtenir une forme non solvatée.

13. Procédé selon l'une quelconque des revendications précédentes, comprenant en outre l'isolement de l'agent anticholinergique, de préférence par :
(i) la formation d'un concentré de la solution comprenant l'anticholinergique à partir du réacteur, de préférence par application de pression réduite ;
(ii) l'ajout d'un antisolvant, dans lequel l'antisolvant est de préférence l'eau pour former une suspension aqueuse ;
(iii) le refroidissement de la suspension aqueuse jusqu'à une température d'environ 0 °C à environ 5 °C ; et
(iv) l'isolement du produit sous une forme cristalline solide, de préférence par filtration.

14. Procédé selon l'une quelconque des revendications précédentes comprenant en outre la micronisation de l'agent anticholinergique.
